# EUROPEAN PATENT SPECIFICATION

(11) **EP 1 037 812 B1**
(45) Date of publication and mention of the grant of the patent: **06.08.2003**
(21) Application number: 98963567.7
(22) Date of filing: 08.12.1998
(51) Int. Cl.: B65D 33/25

(54) **A PACKAGE FOR A VAGINAL RING**
VERPACKUNG FÜR EINEN VAGINALRING
CONDITIONNEMENT POUR ANNEAU VAGINAL

(30) Priority: 12.12.1997 EP 97203910
(43) Date of publication of application: 27.09.2000
(73) Proprietor: Akzo Nobel N.V., 6824 BM Arnhem (NL)
(72) Inventor: FREDERICK, Mervyn, Joseph, NL-5343 EH Oss (NL); RIKKEN, Johannes, Mathias, Gerardus, NL-5345 LJ Oss (NL); VOGELS, Arthur, Gerardus, Johannes, Maria, NL-5345 DM Oss (NL)
(74) Representative: Kraak, Hajo
(86) International application number: EP9808128
(87) International publication number: WO99030976

(56) References cited:
- EP-A- 0 330 786
- DATABASE WPI Section Ch, Week 9542 Derwent Publications Ltd., London, GB; Class A17, AN 95-325308 XP002064582 & JP 07 223653 A (HISAMITSU PHARM CO LTD) 22 August 1995 cited in the application

## Description

The invention pertains to the packaging of a medicated vaginal ring. Such rings are loaded with hormones, usually oestrogens and/or progestagens, and are used for contraception and/or hormone-replacement therapy.

In packaging such vaginal rings, several problems need to be overcome. The package should be capable of maintaining the ring in an uncontaminated state. The package should prevent influences from the environment, such as light, moisture, and micro-organisms, from affecting the vaginal ring. Furthermore, the package should prevent the active substances present in the ring, from leaking to the environment. The latter not only is important during shipping and storing, but in fact throughout the life of the vaginal ring. A vaginal ring is typically used domestically, and could lead to contamination of spots in the house with active substances or contaminated fluids from the vagina, or undesirable contact with children. Correct handling of the ring after use use is therefore essential. On the other hand, for the package to be economically feasible, ready for use, and easy to handle, it should not be of complex design, and it should simply be capable of being in direct contact with the ring, and properly enclosing it and protecting it, nothing more and nothing less.

Such solutions for packaging vaginal rings have not been described in the art. US 4,692,143 pertains to a package containing a vaginal sponge. The disclosure does not relate to packaging a vaginal ring and does not provide any teaching beyond a most general one on using impervious materials.

US 4,620,534 pertains to a sterile package containing an apparatus for inserting an intravaginal article. The disclosure does not pertain to packaging the articles themselves (as these are contained in the insertion apparatus), nor does it relate to vaginal rings. Moreover, the package does not come into direct contact with the intravaginal article. For, apart from the fact that the disclosure pertains to packaging the insertion apparatus containing the article rather than packaging the article itself, it is taught that, if contamination should be a problem, a sleeve is to be provided around the article. Hence, the disclosure is not applicable to the problem of the present invention, which is in seeking a direct packaging solution for a vaginal ring, i.e. including a primary pack that is in direct contact with the ring.

Surprisingly, a type of package, which is of a general class known in itself, has been devised which simultaneously solves all of the above problems. The invention thus resides in the novel use, for packaging a vaginal ring, of a sachet made of a laminate comprising, from the interior side to the exterior side, a layer of a polymeric sealable material, a barrier layer, and a damage protective layer, wherein the layer of the polymeric sealable material is provided, on the inside, with a strip of polymeric material capable of being opened and reclosed, such as a rib and a groove fitting together.

From the viewpoint of avoiding waste of materials, safe handling, and efficient daily practice, the present invention provides an integral packaging solution, which is a major step forward as compared to all kinds of separate boxes or enclosures, or hiding places, that have been or might be devised in order to avoid the active substances of a medicated intravaginal article, and contaminants associated with the used ring, from entering the environment.

It should be noted that, by their very nature, packages which are intended to maintain the quality of aseptically packed or sterile products, lose their purpose after opening, and are disposed of directly. It is a solution quite opposite to what is customary in the art, to provide an aseptic package with means to reclose it. It should further be noted that, in view of the medicated nature of the vaginal ring, it is an essential finding that both the layer of sealable material and the reclosing strip provided on that layer, both being on the inside of the sachet, hence in direct contact with the vaginal ring, be of a pharmaceutically acceptable polymeric material which is compatible with the active substances present in the ring.

Thus, in an unexpectedly simple manner, a package is provided which not only serves the purpose of maintaining the aseptic quality during storing and shipping of the medicated vaginal ring, but also provides the possibility to avoid any influence of the environment on the ring (or vice versa) throughout the life of the ring.

Thus, the basic inventive thought is in the recognition that, contrary to the fundamentals of aseptic and sterilised packaging, a substantial improvement has been achieved by engineering into the package a reclosable feature (reclosing means) for a distinct after use function. The invention, in a preferred embodiment, particularly pertains to the use of a specific type of reclosable package, viz. a laminated foil sachet comprising a rib-and-groove reclosing means, for the aseptic packaging of an intravaginal article.

A package which is in direct contact with a medicated vaginal ring cannot simply be made of a randomly taken material. Several pharmaceutically acceptable materials, mainly plastics, are available which, in the general art of packaging, are used as primary packaging materials. However, one cannot make a suitable sachet thereof. All of such materials take up (absorb) active substance from the medicated article. If a relatively thin layer were used, the absorption capacity is low, but the migration rate will be too high, so the active substances will be able to pass through the layer relatively quickly. A thick layer will facilitate a lower rate of migration, hence a longer period of time needed for permeation through the material, but a thick layer has a higher capacity to take up (in fact, absorb) active substance from the medicated intravaginal article, which eventually leads to an even higher loss of active substance. Further, the package needs to be sufficiently strong in order for it to withstand the damaging effects of handling, transport, and mishandling. It has now been found that this complex of problems can be overcome as a result of the proper choice of the combination of the layers in the package, viz. a layer of a polymeric sealable material, a barrier layer, and a damage protection layer.

Such laminated packages in themselves are known and are in use for all kinds of products, such as foodstuffs and medicines, which sometimes are packed in reclosable sachets. None of the prior art disclosures relates to providing an aseptic package for a vaginal ring.

Thus US 3,827,472 discloses a flexible bag structure for packing foodstuffs and the like, which comprises a polymeric inner layer having an integral, plastics, rib-and-groove reclosing feature. For the e.g. cellophane and paper are mentioned. The disclosure does not pertain to the packaging of vaginal rings, nor to the specific requirement for the layers in the laminate of the present invention.

JP 07/223,653 discloses a laminated packaging bag comprising a seal surrounding the bag and a slideable clip fastener. As a possibility for the primary pack, a laminate of aluminium and polyethylene layers is disclosed. The bag lacks the damage protection layer needed for reliable aseptic or sterile packaging and is used for packaging medicines and foods, rather than vaginal ring.

Several other disclosures exist on laminated packaging films for foods, drugs, granules, or implants, explicitly or implicitly comprising barrier layer and heat seal layers such as aluminium/polyethylene foils. As background disclosures in this respect are mentioned JP 09/142525, EP 709 304, JP 04/253645, JP 04/200549, and BE 690947. None of these disclosures pertains to packaging vaginal rings or to reclosable packages.

Several reclosable bags are known for packaging dangerous fluids, or for other general industrial or household uses. Reference is made to EP 239 319, EP 515 985, US 5,172,980 and US 5,372,429 as background disclosures in this respect.

The person of ordinary skill in the art is capable of putting together the various parts of the present package, including manufacturing the laminated foil and providing the reclosable strip, without undue burden. For, as a general technical concept, the present package for a vaginal ring makes good use of methods and materials that have been known before, e.g. from the aforementioned disclosures. It is particularly in combining the various essential elements of the present invention, that a novel and surprisingly advantageous package for a vaginal ring can be provided.

On the various elements the following can be said.

The layer of sealable material forms the inner layer of the package. It should have the property of being sealable such that, at the edges of the package, two opposite surfaces of the layer can be inseparably adhered together. In principle, all cold or heat sealable materials can be used, with heat-sealable materials being preferred. As the material is in direct contact with the vaginal ring, it must be a pharmaceutically acceptable polymer. Suitable heat-sealable materials include polyethylenes, copolymers or combinations with polyethylene such a polyethylene - ethylene vinyl acetate, polyamides, Surlyns (tradename for a type of polyethylene known as ionomers). It is preferred to use polyethylenes, of all grades, low, medium, and high densities. This not only for the sake of sealing properties, but also because among the polyethylenes are the materials which cause the least absorption of active substances from the vaginal ring. In view of the favourable absorption characteristics, another preferred material is ethylene vinyl acetate copolymer, notably with a relatively low content of vinyl acetate (preferably below 10%, e.g. Evatane 1020 VN3 (9% vinyl acetate content).

Low-density polyethylene (LDPE) is the most preferred sealable material. It has excellent sealing properties, a good melt flow, and it produces a high bond strength at temperatures which are easily achieved by normal heat sealing machines, which operate on a bar seal or roller seal principle. The good melt flow ensures a good spread of the sealing layer and a good fusion of the contacting surfaces forming the seal, to create a hermetic seal which is impervious to microbes and bacteria. Once a seal has been obtained, it is virtually impossible to separate the materials forming the bond. This is essential in order to ensure that once the ring has been packed in the sachet, the ring will remain of low bioburden from the moment of packaging and during all stages of handling and transport until the pack is opened for removing the ring. The term "bioburden" is used to refer to both the level of micro-organisms or bacteria present as well as to the level of contamination by micro-organisms that may be caused. Moreover, LDPE was found to be a surprisingly excellent choice for being in direct contact with the vaginal ring. This material does not promote migration of active ingredients from the ring into the sealing layer and the degree of absorption of the active materials has been found to be virtually negligible.

The thickness of the layer of sealable material will generally be of from 10 to 80 µm, and preferably 30-60 µm. As a rule, a thickness of below 10 µm will give problems with the sealability, while a thickness of more than 80 µm results in too large a reservoir for absorption of active substances from the ring.

The barrier layer can in principle be made of any metal foil-containing material, such as aluminium foil, tin foil, gold foil, metallized coatings which may be on plastics films, and the like. Also suitable are coatings of oxides such as aluminium oxides, silicon oxides, and nitrides on plastic substrates such as polyester films and the like. The barrier layer prevents any possibility of active materials diffusing through the layers of the primary pack.

Aluminium foil is the most preferred barrier layer. This foil has optimal properties with regard to impermeability to light, micro-organisms, gases, vapours, and active substances, thus providing the best possible protection of the vaginal ring against light and maintaining a low bioburden until use. Aluminium foil also is a surprisingly favourable choice as far as preventing potential loss of active material from the ring to the outside of the pack, as well as avoiding the risk of contamination of the outside of the primary package. Thus, the stability and consistency of the ring is preserved, and anyone handling the sachet in which the ring is stored, is prevented from inadvertently coming into contact with the active ingredients of the ring.

The thickness of the barrier layer will generally be of from 7 to 25 µm for metal foils, of from 1 to 2 µm of metal for metallized coatings on plastic foils, and of from 0.1 to 1 µm of oxide for oxide coatings.

The damage protective layer can be made from all sheet materials that have sufficient strength and are sufficiently tear-resistant, such as polypropylene, polyamide, cellophane, paper. The most preferred damage protective layer is made of polyester (polyethylene terephthalate).

It should be noted that the laminated packages known in the art do not contain such an additional external damage protective layer. If the state of the art metal foil/plastics sealing layer laminated packages are to be used for products where the level of bioburden must be guaranteed to be low, the laminate used should be unduly thick. If a thick metal layer were used, the package would become difficult to produce on regular machines. If a thick plastics sealing layer were used, the aforementioned problem of absorption capacity of active substances from the ring would be incurred to an unnecessary and unacceptable extent, and the initial opening the sachet to remove the ring would not be easy and would requiring the use of a helping device such as a pair of scissors etc. It is by the very measure of including a separate damage protective layer, which can be readily torn open by hand, that all of the requirements for the package are most elegantly satisfied.

The thickness of the damage protective layer will generally be of from 10 to 30 µm for plastics and from 30 to 60 g/m² for paper.

The reclosable strip can be based on any opening-closing mechanism in which one or more protrusions are associated with corresponding indentations. Such strips are known in the art. The best example thereof is a rib and corresponding groove along the entire length of the strip. For the rib, and thus also for the groove, several different profiles can be chosen. It is essential that the reclosable strip is made of a polymeric, or polymer-based, or elastomeric material, e.g. high-density polyethylene, polypropylene, and the like, all of which must be of acceptable pharmaceutical grade. This is not only for manufacturing reasons (in principle it is standard technology to provide a plastic package with a plastic reclosable strip), but also because the strip, as an essential component of the primary packaging materials, is on the inside of the sachet and thus the edges thereof may come into contact with the vaginal ring. The reclosable strip, being of relatively substantial mass with respect to the potential for absorption of active constituents from the vaginal ring, is most preferably chosen of high-density polyethylene, as this material displays a significantly low level of absorption of active substances from the ring, and upon such absorption even ceases to absorb further.

The production of the walls of the sachet may be achieved via a conventional laminating process. The reclosable strip can made by continuous extrusion, but may also be manufactured using other processes such as moulding, injection moulding, and the like. Making an assembly of the laminated package and the reclosable strip can be done by conventional processes such as heat sealing, ultrasonic welding, or by employing adhesives. After being provided with the reclosable strip and after being cut and formed into a sachet of the appropriate dimensions, the package is ready to contain a vaginal ring, after which the ring is inserted and the sachet is sealed under aseptic conditions.

The most preferred, as an excellent, though simple packaging solution, is the use of the type of sachet known for packaging chewing gum and the like, which has the aforementioned closing means and in which the successive layers are 35-45 µm (more preferably 40 µm ± 4 µm LDPE (inside) as a sealing layer, 8-10 µm (more preferably 9 µm ± 0.7 µm) aluminium foil as a barrier layer, and 10-14 µm (moe preferably 12 µm ± 1.2 µm) PET (outside) as a damage protective layer. The closing strip in these preferred sachets is made of LDPE and has a single rib-groove profile.

The invention is hereinafter further explained with reference to the drawings.

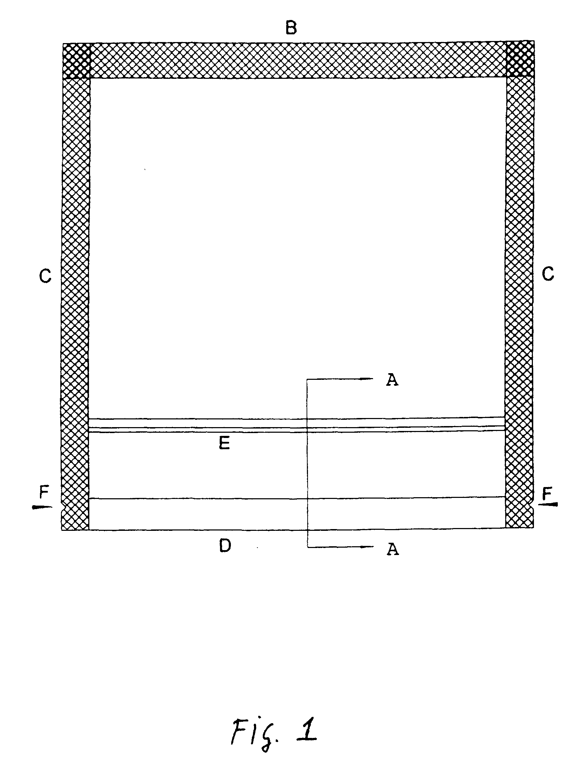

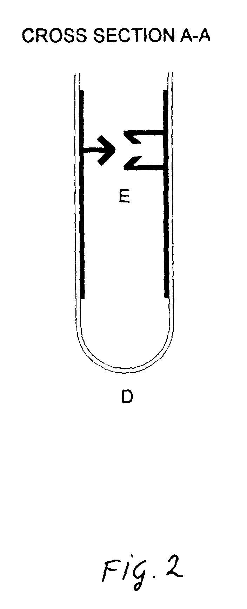

FIG. 1 is a front-side view of a sachet of the invention, with the side to be opened positioned downwards. FIG. 2 is a cross-section along the line A-A in FIG. 1.

The sachet in FIG.1 is shown to have a sealed edge B, wherein the opposite inner layers have been sealed together (normally after the ring has been inserted), side seals C, which are formed by heat sealing opposite layers of the material prior to filling, a folded section of the laminate D, a reclosable strip E, and notches F. These notches serve to enhance the easy opening of the otherwise damage-protected, hence strong, material at a position beyond the part that can be opened and re-closed by means of the reclosable strip C.

In FIG. 2 are depicted the fold D, and the reclosable strip E, the latter comprising a rib G and a groove H.

## Claims

1. An aseptic package enclosing an intravaginal article, the package being made of a laminate comprising, from the interior side to the exterior side, a layer of a sealable material, a barrier layer, and a damage protective layer, wherein the package comprises means for reclosing it after opening, said means being in the form of a strip of polymeric material.

2. A package according to claim 1, **characterised in that** the sealable material is a heat-sealable polymer.

3. A package according to claim 1 or 2, **characterised in that** the barrier layer is a metal foil.

4. A package according claims 2 and 3, **characterised in that** the heat-sealable polymer is polyethylene, the metal foil is aluminium, and the damage protective layer is a polyester film.

5. The use of a sachet made of a polyethylene - aluminium foil laminate and comprising a plastic rib-and-groove reclosing means, for the aseptic packaging of an intravaginal article, the laminate being provided with a polyester outer layer.

6. A use according to claim 5, **characterized in that** the laminate comprises a low-density polyethylene inner layer of 35-45 µm, an aluminium foil barrier layer of 8-10 µm, and a PET protective outer layer of 10-14 µm.

## Patentansprüche

1. Eine aseptische Verpackung für einen intravaginalen Artikel, wobei die Verpackung aus einem Laminat hergestellt ist, welches von der Innenseite zur äusseren Seite hin gesehen eine Schicht aus einem dichtenden Material, eine Sperrschicht und eine vor Beschädigungen schützende Schicht umfasst, wobei die Verpackung Mittel zum Wiederverschliessen nach ihrer Öffnung umfasst, wobei die besagten Mittel die Gestalt eines Streifens aus Polymermaterial aufweisen.

2. Verpackung nach Anspruch 1, **dadurch gekennzeichnet, dass** das dichtende Material ein wärmedichtendes Polymer ist.

3. Verpackung nach Anspruch 1 oder 2, **dadurch gekennzeichnet, dass** die Sperrschicht eine Metallfolie ist.

4. Verpackung nach Anspruch 2 und 3, **dadurch gekennzeichnet, dass** das wärmedichtende Polymer Polyäthylen ist, die Metallfolie Aluminium und die vor Beschädigungen schützende Schicht ein Polyesterfilm.

5. Verwendung eines Beutels aus einem Polyäthylen-Aluminium-Folien-Laminat und umfassend ein Rippen-und-Nut-Wiederverschlussmittel aus Kunststoff zur aseptischen Verpackung eines intravaginalen Artikels umfasst, wobei das Laminat mit einer äusseren Polyesterschicht versehen ist.

6. Verwendung nach Anspruch 5, **dadurch gekennzeichnet, dass** das Laminat eine innere Schicht eines Polyäthylens geringer Dichte von 35 bis 45 µm, eine Aluminiumfolien-Sperrschicht von 8 bis 10 µm und eine schützende äussere Schicht aus PET mit 10 bis 14 µm umfasst.

## Revendications

1. Emballage aseptique renfermant un article intravaginal, l'emballage étant constitué d'un laminé comprenant, depuis l'intérieur vers l'extérieur, une couche d'un matériau scellable, une couche de barrière et une couche de protection contre l'endommagement, dans lequel l'emballage comprend des moyens pour le refermer après ouverture, lesdits moyens étant de la forme d'une bande de matériau polymérique.

2. Emballage selon la revendication 1, **caractérisé en ce que** le matériau scellable est un polymère thermoscellable.

3. Emballage selon la revendication 1 ou 2, **caractérisé en ce que** la couche de barrière est une feuille de métal.

4. Emballage selon les revendications 2 et 3, **caractérisé en ce que** le polymère thermoscellable est du polyéthylène, la feuille de métal est de l'aluminium et la couche de protection contre l'endommagement est un film polyester.

5. Utilisation d'un sachet constitué d'un laminé de polyéthylène - feuille d'aluminium et comprenant des moyens de refermeture à nervure et rainure en plastique, pour le conditionnement aseptique d'un article intravaginal, le laminé présentant une couche externe de polyester.

6. Utilisation selon la revendication 5, **caractérisée en ce que** le laminé comprend une couche interne de polyéthylène basse densité de 35-45 µm, une couche de barrière en feuille d'aluminium de 8-10 µm et une couche externe de protection PET de 10-14 µm.
